## Europäisches Patentamt

## European Patent Office

## Office européen des brevets

(19)

(11) Veröffentlichungsnummer: **0 097 264**

**A1**

(12) # EUROPÄISCHE PATENTANMELDUNG

(21) Anmeldenummer: **83105340.0**

(22) Anmeldetag: **30.05.83**

(51) Int. Cl.³: **A 61 N 1/08**
**A 61 N 1/36, G 08 C 17/00**
**H 04 B 1/59**

(30) Priorität: **03.06.82 DE 3220930**

(43) Veröffentlichungstag der Anmeldung:
**04.01.84 Patentblatt 84/1**

(84) Benannte Vertragsstaaten:
**DE FR GB NL SE**

(71) Anmelder: **Siemens-Elema AB**
**Röntgenvägen 2**
**S-171 95 Solna 1(SE)**

(84) Benannte Vertragsstaaten:
**SE**

(71) Anmelder: **SIEMENS AKTIENGESELLSCHAFT**
**Postfach 22 02 61**
**D-8000 Munchen 22(DE)**

(84) Benannte Vertragsstaaten:
**DE FR GB NL**

(72) Erfinder: **Vock, Josef**
**Raettarvägen 2**
**S-171 52 Solna(SE)**

(54) **Zweiwegkommunikationssystem zwischen einem implantierten elektrischen Stimulator und einer externen Kontrolleinheit.**

(57) Um bei einem Zweiwegkommunikationssystem zwischen einem implantierten elektrischen Stimulator (1) und einer externen Kontrolleinheit (5) die Anforderungen an die Dynamik des Empfängerteiles des Stimulators (1) bei hoher Uebertragungsqualität senken und außerdem die Sendeleistung der Kontrolleinheit (5) zu minimieren, ist diese erfindungsgemäß mit einer ersten Anordnung (16) versehen, mit derein der empfangenen Signalstärke proportionales Signal erzeugt wird. Dieses Signal steuert über eine Regeleinrichtung (24,25) die Sendeleistung der Kontrolleinheit (5).

./...

EP 0 097 264 A1

SIEMENS AKTIENGESELLSCHAFT
Berlin und München

Unser Zeichen
VPA 82 P 7309 E

**Zweiwegkommunikationssystem zwischen einem implantierbaren elektrischen Stimulator und einer externen Kontrolleinheit**

Die Erfindung betrifft ein Zweiwegkommunikationssystem für die drahtlose Nachrichtenübertragung zwischen einem implantierbaren elektrischen Stimulator mit einem Sende/Empfangsteil und einer externen Kontrolleinheit mit ebenfalls einem Sende/Empfangsteil, wobei die Nachrichtenübertragung wenigstens von der Kontrolleinheit zum Stimulator mit Rückmeldung erfolgt. Derartige Zweiwegkommunikationssysteme finden beispielsweise bei implantierbaren Herzschrittmachern Anwendung, bei denen mit Hilfe der externen Kontrolleinheit verschiedene Parameter des Herzschrittmachers wie beispielsweise die Frequenz, die Ausgangsspannung der Stimulierungsimpulse oder auch die Empfindlichkeit eingestellt werden können. Durch Rückmeldung wird dabei sichergestellt, dass die von der Kontrolleinheit übermittelte Information vom Stimulator korrekt empfangen worden ist. Weiterhin ist es bei derartigen Systemen unter Umständen möglich, vom implantierten Stimulator registrierte Messgrössen wie beispielsweise den Schwellwert eines Herzschrittmachers oder ein internes EKG an die Kontrolleinheit zu senden.

Um bei derartigen bekannten Zweiwegkommunikationssystemen eine einwandfreie Nachrichtenübermittlung sicherzustellen, müssen hohe Anforderungen insbesondere an die Dynamik des Empfängerfilters in dem implantierten Stimulator gestellt werden. Ist beispielsweise der Stimula-

Gdl 1 Een / 27.5.1982

tor mit einer Empfängerspule ausgerüstet, so kann die darin induzierte Spannung in Abhängigkeit vom Abstand des Senders erheblich variieren. In der Praxis durchaus vorkommende Abstände zwischen Stimulator und Kontrolleinheit liegen zwischen 1 und 15 cm und führen bei konstanter Sendeleistung in der Kontrolleinheit zu 100 bis 10000 mV induzierter Spannung in der Empfängerspule. Insbesondere bei bitorientierten Kommunikationssystemen mit einer festvorgegebenen Empfindlichkeit eines Schwellwertdetektors im Empfängerteil des implantierten Stimulators können hohe induzierte Spannungen und damit verbundene nachfolgende lange Nachschwingverläufe über der Spule zu einer Symbolinterferenz führen. Dadurch entsteht ein erhebliches Risiko für eine fehlerhafte Signalerkennung.

Der vorliegenden Erfindung liegt die Aufgabe zugrunde, bei einem Zweiwegkommunikationssystem der eingangs genannten Art die Forderung nach hoher Dynamik des Empfängerteils in dem Stimulator wesentlich zu senken. Darüberhinaus soll auch die aufzubringende Leistung im Sendeteil der Kontrolleinheit minimiert werden.

Diese Aufgabe wird erfindungsgemäss dadurch gelöst, dass die externe Kontrolleinheit eine erste Anordnung für die Erzeugung eines der empfangenen Signalstärke entsprechenden Signales sowie eine weitere Anordnung aufweist, die in Abhängigkeit davon die Sendeleistung der externen Einheit regelt. Erfindungswesentlich ist also, dass das Zweiwegkommunikationssystem Anordnungen enthält, die automatisch die Sendeleistung der Kontrolleinheit auf solche Weise regeln, dass das Empfangsteil in dem implatierten Stimulator, insbesondere das Empfangsfilter, stets ein gutes Signalniveau zur weiteren Verarbeitung vorfindet. Dabei wird mit Vorteil die Zweiwegekommunikation und die Tatsache ausgenutzt, dass zumindestens die Nachrichtenübertragung von der Kontrolleinheit zum Stimulator mit Rückmeldung erfolgt (handshake-Verfahren). Die Stärke des von der Kontrolleinheit wieder empfangenen Signales enthält dazu alle notwendige Information.

In einer vorteilhaften Weiterbildung der Erfindung ist vorgesehen, dass als erste Anordnung ein bekannter automatischer Regelverstärker (AGC-Verstärker) dient, dessen Rückkopplungssignal das Mass für die empfangene Signalstärke darstellt. Im AGC-Verstärker wird de facto eine Signalmessung vorgenommen, die in einer AGC-Spannung resultiert. Diese Spannung enthält die gewünschte Information über den Abstand respektive den effektiven Kopplungsfaktor zwischen Sender und Empfänger. Die Sendeleistung des Senders in der Kontrolleinheit kann mit Hilfe dieser AGC-Spannung gesteuert werden.

Für die digitale Nachrichtenübertragung ist es mit Vorteil ebenso möglich, dass die weitere Anordnung die Breite der zu übertragenen Impulse steuert.

Eine zusätzliche Möglichkeit, die Uebertragungsqualität des Kommunikationssystems zu verbessern, besteht darin, dass die Kontrolleinheit ein Glied zum Bestimmen der Fehlerfrequenz im Antwortsignal des implantierten Stimulators aufweist und das die Sendeleistung der Kontrolleinheit zusätzlich durch diese Fehlerfrequenz geregelt ist.

Anhand eines Blockschaltbildes wird im folgenden ein Ausführungsbeispiel der Erfindung näher beschrieben und erläutert.

0097264

82 P 7 3 0 9 E

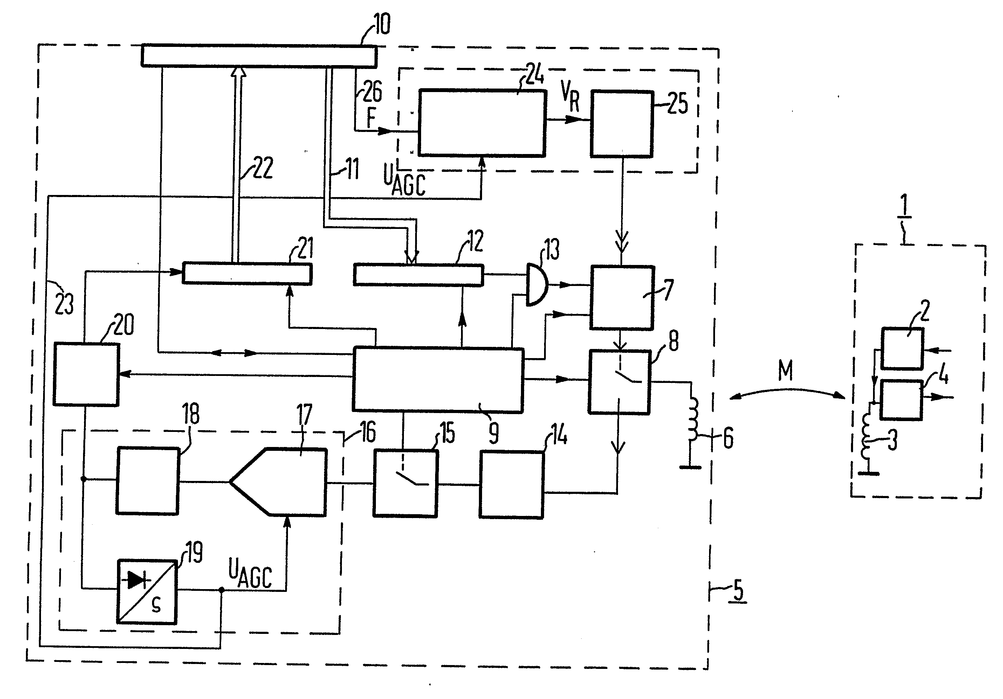

Die einzige Figur zeigt dabei im rechten Teil schematisch als Block angedeutet einen implantierten elektrischen Stimulator 1, wie er beispielsweise aus dem SIEMENS ELEMA-Prospekt ME 372/5432.101 oder aus der US PS 4223679 bekannt ist. Von diesem Stimulator ist lediglich ein Sender 2, eine Sende- bzw. Empfangsspule 3 und ein Eingangsfilter 4 näher dargestellt.

Im linken Teil der Figur ist, wiederum als Block, die nichtimplantierte Kontrolleinheit 5 dargestellt, die im folgenden näher beschrieben wird. Die Kontrolleinheit enthält ebenfalls eine Sende/Empfangsspule 6 sowie eine Sendestufe 7. Zwischen Spule und Sendestufe ist ein elektronischer Sende/Empfangsumschalter 8 geschaltet, der über eine Zeitablaufsteuerung 9 betätigt wird. In der dargestellten Figur befindet sich dieser Schalter 8 in der Sendestellung.

Weiterhin enthält die Kontrolleinheit 5 eine Anwendeschnittstelle 10, über die Befehle zur Weiterleitung an den implantierten Stimulator eingegeben und andererseits Informationen von dem Stimulator entnommen werden können. Von der Anwendeschnittstelle 10 wird die zu übertragende Information über eine Leitung 11 beispielsweise parallel auf ein Datensenderegister 12 gegeben und von dort über einen Pulspositionsmodulator 13 auf die Sendestufe 7. Sowohl Datensenderegister, Pulspositionsmodulator und Sendestufe werden von der Zeitablaufsteuerung 9 zeitlich koordiniert. Vom Datensenderegister 12 aus wird die zu übertragende Information seriell weitergeleitet.

Wenn der elektronische Sende/Empfangsumschalter 8 auf Empfang umschaltet, so wird die in der Spule 6 induzierte Spannung über einen Vorverstärker 14 und einen ebenfalls über die Zeitablaufsteuerung betätigten Austaster 15 (elektronischer Blancing-Schalter) auf eine automatische Verstärkungsregelungsstufe 16 gegeben, wie sie im Prinzip beispielsweise bei Rundfunk- oder Fernsehempfängern angewendet wird, um unterschiedliche Empfangsfeldstärken auszugleichen. Im vorliegenden Beispiel besteht diese Stufe aus einem AGC-Verstärker (Automatic Gain Controler) 17, dessen Ausgang über ein Bandpassfilter 18 und einen Vollweggleichrichter mit Tiefpassfilter 19 wieder auf den Verstärker 17 rückgekoppelt ist. Das Tiefpassfilter sorgt dafür, dass für die Zeit, in der kein Eingangssignal an der Regelstufe 16 anliegt, das Rückkoppelsignal unverändert erhalten bleibt. Die Spannung $U_{AGC}$ stellt praktisch einen Wert für den effektiven Kopplungsfaktor zwischen den Spulen 3 und 6 bzw. für das empfangene Nutzsignal dar.

Weiterhin ist der Ausgang des Bandpassfilters 18 über ein beispielsweise aus dem Buch von Göran Einarsson "Detekteringsteori, datatransmission och pulskomunikation" bekanntes Datenrekonstruktionsfilter 20 auf ein Datenempfangsregister 21 geschaltet, von dem die empfangene Information über eine Leitung 22 parallel zur Anwenderschnittstelle 10 geleitet wird.

Die Rückkopplungsspannung für den AGC-Verstärker 17 wird über eine Leitung 23 als Steuergrösse einem Regelspannungsgenerator 24 zugeführt, dessen Ausgangsspannung $V_R$ ein Stellglied 25 für die Sendeleistung der Sendestufe 7 ansteuert.

Bereits durch diese Massnahmen ist es möglich, die Sendeleistung der Sendestufe 7 stets auf einen Wert zu regeln, so dass eine optimale Nachrichtenübertragung von der Kontrolleinheit 5 zum implantierten Stimulator 1 ge-

0097264

82 P 7 3 0 9 E

geben ist.

Weiterhin kann in der Anmelderschnittstelle 10 die Fehlerwahrscheinlichkeit zwischen dem gesendeten und im "handshake-Verfahren" empfangenen Antwortsignal ermittelt werden und über eine Leitung 26, wie durch den Pfeil F angedeutet, ebenfalls als Steuergrösse auf den Regelspannungsgenerator 24 gegeben werden. Die Fehlerwahrscheinlichkeit dient dabei als zusätzliche Steuergrösse für die Bestimmung der Sendeleistung.

Neben der Sicherstellung einer guten Nachrichtenübertragungsqualität wird durch diese Regelung der Sendeleistung auch noch erreicht, dass der Energieverbrauch der peripheren Kontrolleinheit verringert wird, was insbesondere bei der Anwendung in einem "Holder-Monitor" von Interesse ein kann.

5 Ansprüche
1 Figur

Patentansprüche

1. Zweiwegkommunikationssystem für die drahtlose Nach-richtenübertragung zwischen einem implantierbaren elek-trischen Stimulator mit einem Sende/Empfangsteil und einer externen Kontrolleinheit mit ebenfalls einem Sende/Empfangsteil, wobei die Nachrichtenübertragung wenigstens von der externen Einheit zum Stimulator mit Rück-meldung erfolgt (handshake - Verfahren), d a d u r c h g e k e n n z e i c h n e t, dass die externe Kontroll-einheit (5) eine erste Anordnung (16) für die Erzeugung eines der empfangenen Signalstärke entsprechenden Signals ($U_{AGC}$) sowie eine weitere Anordnung (24,25) aufweist, die in Abhängigkeit davon die Sendeleistung der externen Einheit (5) auf einen eine hohe Signalübertragungsqualität ergebenden Wert regelt.

2. Zweiwegkommunikationssystem nach Anspruch 1, d a -d u r c h  g e k e n n z e i c h n e t , dass als erste Ordnung eine automatische Verstärkungsregelungsstufe (17) dient, deren Rückkopplungssignal ($U_{AGC}$) das Mass für die empfangene Signalstärke darstellt.

3. Zweiwegkommunikationssystem nach Anspruch 1 oder 2, d a d u r c h  g e k e n n z e i c h n e t , dass die weitere Anordnung (24,25) die Versorgungsspannung des Senders (7) in der externen Einheit (5) und somit die Sendeleistung steuert.

4. Zweiwegkommunikationssystem für die digitale Nachrichtenübertragung nach Anspruch 1 oder 2, d a -d u r c h  g e k e n n z e i c h n e t , dass die wei-tere Anordnung (24,25) mittels Pulsbreitenmudulation die Leistung des Senders (7) in der externen Einheit (5) steuert.

5. Zweiwegkommunikationssystem nach einem der Ansprüche 1,2 oder 4, d a d u r c h   g e k e n n z e i c h n e t dass die Kontrolleinheit (5) ein Glied zum Bestimmen der Fehlerfrequenz im Antwortsignal des implantierten Stimulators (1) aufweist und das die Sendeleistung der externen Kontrolleinheit (5) zusätzlich durch diese Fehlerfrequenz (F) geregelt ist.

1/1

Europäisches Patentamt

**EUROPÄISCHER RECHERCHENBERICHT**

**0097264**
Nummer der Anmeldung

| EINSCHLÄGIGE DOKUMENTE | | | EP 83105340.0 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
| D,A | US - A - 4 223 679 (SCHULMAN)  -- | | A 61 N 1/08 <br> A 61 N 1/36 |
| A | US - A - 3 925 782 (ANDERL) <br> * Totality * <br> ---- | 1-3 | G 08 C 17/00 <br> H 04 B 1/59 |
| | | | **RECHERCHIERTE SACHGEBIETE (Int. Cl. ³)** |
| | | | A 61 N <br> G 08 C <br> H 04 B |

Der vorliegende Recherchenbericht wurde für alle Patentansprüche erstellt.

| Recherchenort | Abschlußdatum der Recherche | Prüfer |
|---|---|---|
| WIEN | 27-09-1983 | NEGWER |